# EUROPEAN PATENT SPECIFICATION

(11) **EP 2 974 646 B1**
(45) Date of publication and mention of the grant of the patent: **06.12.2017**
(21) Application number: 14788382.1
(22) Date of filing: 28.03.2014
(51) Int. Cl.: A61B 1/00

(54) **ENDOSCOPE ADAPTER AND ENDOSCOPE**
ENDOSKOPADAPTER UND ENDOSKOP
ADAPTATEUR D'ENDOSCOPE ET ENDOSCOPE

(30) Priority: 22.04.2013 JP 2013089765
(43) Date of publication of application: 20.01.2016
(73) Proprietor: Olympus Corporation, Tokyo 192-8507 (JP)
(72) Inventor: MURAYAMA, Masahiko, Tokyo 192-8507 (JP)
(74) Representative: Schicker, Silvia
(86) International application number: PCT/JP2014/059162
(87) International publication number: WO 2014/174989

(56) References cited:
- EP-A1- 1 882 442
- JP-A- 2001 057 962
- JP-A- 2003 310 540
- JP-U- S 649 607

## Description

### Technical Field

The present invention relates to an adaptor for endoscopes, and an endoscope, and specifically relates to an adaptor for endoscopes that is attachable to a treatment instrument insertion port of an endoscope, and an endoscope.

### Background Art

Conventionally, endoscopes have been widely used in, e.g., a medical field. Endoscopes are each used for performing, e.g., an examination by inserting an insertion portion into an examination target and displaying, on a display apparatus, an endoscopic image picked up by an image pickup device provided in a distal end portion of an insertion portion or obtained via an image guide inserted inside the insertion portion.

Also, there are various types of adapters for endoscopes. One of the adaptors for endoscopes is an adaptor that can be fitted to a treatment instrument insertion port in an endoscope, the adaptor enabling water to be fed while a treatment instrument is inserted. Use of such adaptor enables high frequency treatment to be performed while a high-frequency treatment instrument is inserted into a treatment insertion channel in an insertion portion and a conductive liquid such as saline is being supplied to a site to be treated.

When a high frequency treatment is performed while water is being fed, if the adaptor is made of a metal, for example, high-frequency current flows to the metal adaptor via the saline, a user of the endoscope may touch a metal-exposed portion and suffer a burn. Thus, for example, as disclosed in Japanese Patent Application Laid-Open Publication No. 2001-57962, an adaptor in which, e.g., a cock body is formed by an electrical insulating resin material or an outer face of a metal member is covered by an electrical insulating member to prevent a user of an endoscope from touching a metal-exposed portion and suffering a burn has been proposed.

However, forming, e.g., a cock body of an adaptor from an electrical insulating resin material to prevent such a burn injury results in decrease of the strength of the duct of the adaptor itself. To increase the strength of the duct of the adaptor itself, it is generally desirable to configure the duct itself with a member made of metal. In this case, a possible way to cover the member made of metal with a resin is to employ an integral insert molding method using metal and resin. This method however can cause a crack in the resin due to inner residual stress in the resin after being molded.

An alternative way is to cause the resin to adhere to the metal with an adhesive so as to cover each member made of metal with electrical insulating resin. However, fixation by means of only an adhesive can cause detachment of the resin from the metal due to deterioration of the adhesive.

JP 2003-310540A discloses an endoscope adapter comprising: an endoscope attachment part which is attached to a treatment tool insertion opening of the endoscope; and a conduit member having a first port part which communicates with the treatment tool insertion opening, and a second port part which communicates with the treatment tool insertion opening.

An objective of the present invention is to provide an adaptor for an endoscope and an endoscope capable of ensuring safety at the time of use of high-frequency current without decreasing the strength of the duct of the adaptor itself.

### Disclosure of Invention

### Means for Solving the Problem

An adaptor for an endoscope according to an aspect of the present invention includes the features of claim 1. An adaptor may include: an endoscope attachment portion to be attached to a treatment instrument insertion port of the endoscope; a metal duct member including a first port portion that communicates with the treatment instrument insertion port, and a second port portion that communicates with the treatment instrument insertion port; a first sheathing member including an insulating member, the first sheathing member covering at least a part of an outer surface of the first port portion and including an extension portion that extends so as to spread toward a proximal end side; and a second sheathing member including an insulating member, the second sheathing member covering at least a part of an outer surface of the second port portion, and covering the extension portion of the first sheathing member so as to prevent the sheathing member from moving to a distal end side of the first port portion and coming off.

An endoscope according to an aspect of the present invention includes an adaptor for an endoscope according to the present invention.

### Brief Description of the Drawings

Fig. 1 is a configuration diagram illustrating a configuration of an endoscope according to an embodiment of the present invention;
Fig. 2 is a perspective view illustrating an outer appearance of an adaptor 6 according to the embodiment of the present invention;
Fig. 3 is a front view illustrating the outer appearance of the adaptor 6 according to the embodiment of the present invention;
Fig. 4 is a half cross-sectional view along an axis direction of the adaptor 6 according to the embodiment of the present invention;
Fig. 5 is a cross-sectional view along line V-V in Fig. 4;
Fig. 6 is a perspective view of a duct body 21 included in a port portion 11 according to the embodiment of the present invention;
Fig. 7 is a front view of the duct body 21 according to the embodiment of the present invention;
Fig. 8 is a plan view of the duct body 21 according to the embodiment of the present invention;
Fig. 9 is a half cross-sectional view along line IX-IX in Fig. 8;
Fig. 10 is a front view of a duct member 22 included in a port portion 12 according to the embodiment of the present invention;
Fig. 11 is a half cross-sectional view of the duct member 22 along line XI-XI in Fig. 10;
Fig. 12 is a perspective view of a water feeding duct cover 31 according to the embodiment of the present invention as viewed obliquely from above;
Fig. 13 is a perspective view of the water feeding duct cover 31 according to the embodiment of the present invention as viewed obliquely from underneath;
Fig. 14 is a front view of the water feeding duct cover 31 according to the embodiment of the present invention;
Fig. 15 is a half cross-sectional view along line XV-XV in Fig. 14;
Fig. 16 is a perspective view of a duct body cover 41 according to the embodiment of the present invention as viewed obliquely from above;
Fig. 17 is a front view of the duct body cover 41 according to the embodiment of the present invention;
Fig. 18 is a plan view of the duct body cover 41 according to the embodiment of the present invention;
Fig. 19 is a right side view according to the embodiment of the present invention;
Fig. 20 is an exploded and assembly view for describing a method for assembling the adaptor 6 according to the embodiment of the present invention; and
Fig. 21 is a cross-sectional view of an adaptor 6A according to a modification of the adaptor 6 according to the embodiment of the present invention, along line V-V in Fig. 4.

### Best Mode for Carrying Out the Invention

An embodiment of the present invention will be described below with reference to the drawings.

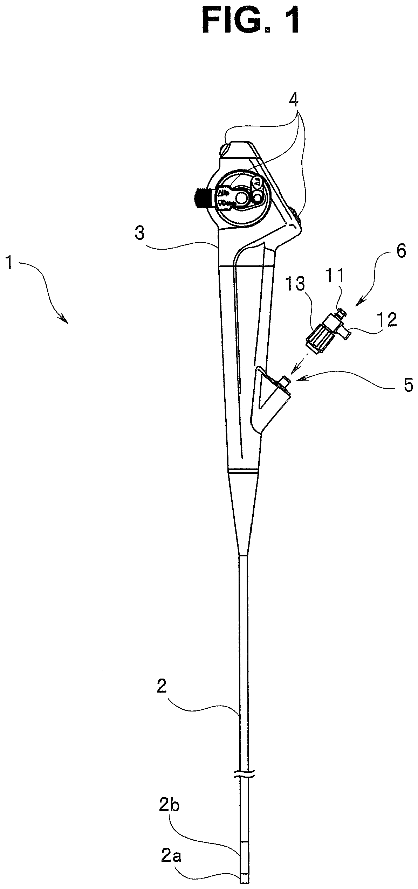

Fig. 1 is a configuration diagram illustrating a configuration of an endoscope according to the present embodiment. As illustrated in Fig. 1, an endoscope 1 includes an elongated insertion portion 2 having flexibility, and an operation portion 3.

Although not illustrated, at a distal end portion 2a of the insertion portion 2, an illumination window for applying illuminating light to a site to be examined, an observation window for receiving reflected light from the site to be examined, and a treatment instrument opening are provided.

Also, at the operation portion 3, a bending knob for bending a bending portion 2b provided on the proximal end side of the distal end portion 2a, and a plurality of operation members 4 for various functions such as a release button for shooting are provided. Furthermore, in the operation portion 3, a treatment instrument insertion port 5 is provided.

Inside the insertion portion 2, a treatment instrument insertion channel (not illustrated) is formed, and an end of the treatment insertion channel (not illustrated) communicates with the treatment instrument opening at the distal end portion 2a of the insertion portion 2, and the other end of the treatment insertion channel communicates with the treatment instrument insertion port 5 provided in the operation portion 3.

An image signal to be processed, which has been picked up by an image pickup device in the distal end portion of the insertion portion 2, is transmitted to a processor apparatus via a non-illustrated universal cable, and an endoscopic image is displayed on a monitor connected to the processor apparatus.

Furthermore, a surgeon can perform any of various treatments on a site to be treated by inserting a treatment instrument into the treatment instrument insertion port 5 of the endoscope 1 and making the treatment instrument project from the treatment instrument opening in the distal end portion 2a via the treatment insertion channel.

In particular, when the surgeon performs a high frequency treatment while a high-frequency treatment instrument is inserted through the treatment insertion channel of the insertion portion 2 and a conductive liquid such as saline is being supplied to a site to be treated, the surgeon fits an adaptor 6 to the treatment instrument insertion port 5.

Also, in order to perform an observation while water is being fed without performing a high frequency treatment, the surgeon may fit the adaptor 6 to the treatment instrument insertion port 5.

The adaptor 6 includes two port portions 11 and 12, and an endoscope attachment portion 13 for attaching the adaptor 6 to the treatment instrument insertion port 5 of the endoscope 1. The port portion 11 is a forceps port for inserting a high-frequency treatment instrument, and the port portion 12 is a water feeding port for supplying a liquid.

Note that although, here, a conductive liquid such as saline is indicated as an example of a liquid to be supplied to the port portion 12 in order to perform a high frequency treatment, a liquid that hardly conducts electricity may be used as a liquid to be supplied to the port portion 12 in order to perform a high frequency treatment.

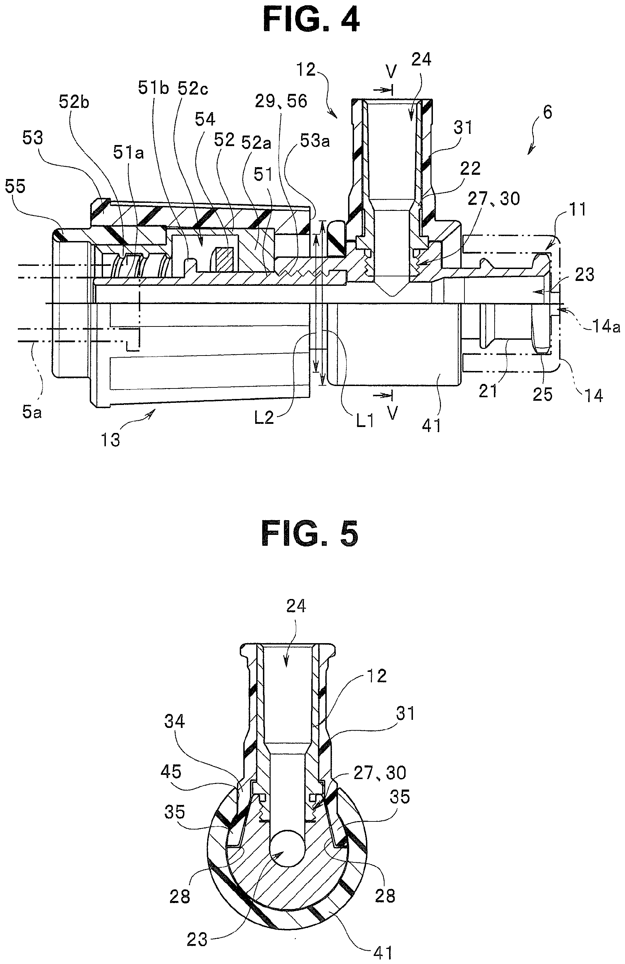

A forceps plug 14 (Fig. 4), which includes an insulating member, can be attached to a distal end portion of the port portion 11, and can cover a metal fitting portion on the distal end side of the port portion 11. In a center portion of the forceps plug 14, a hole 14a is formed, and a high-frequency treatment instrument can be inserted through the hole 14a. In other words, the port portion 11 is configured in such a manner that the forceps plug 14, which is a plug member including an insulating member, can be attached to the distal end portion.

Also, a water feeding tube in a water feeding apparatus that supplies a liquid such as saline is connected to the port portion 12. The endoscope attachment portion 13 is a mechanism section for attaching the adaptor 6 to the treatment instrument insertion port 5 of the endoscope 1.

A high-frequency treatment instrument is inserted into the treatment insertion channel from the port portion 11 with the endoscope attachment portion 13 of the adaptor 6 attached and fixed to the treatment instrument insertion port 5, a distal end portion of the high-frequency treatment instrument is made to project from the treatment instrument opening of the distal end portion 2a, and water is fed into the treatment insertion channel from the port portion 12, enabling the surgeon to perform a high frequency treatment while the liquid is being supplied to a site to be treated. In other words, the adaptor 6 is an adaptor that can be fitted to the treatment instrument insertion port 5 of the endoscope 1, and enables water feeding while a high-frequency treatment instrument is inserted.

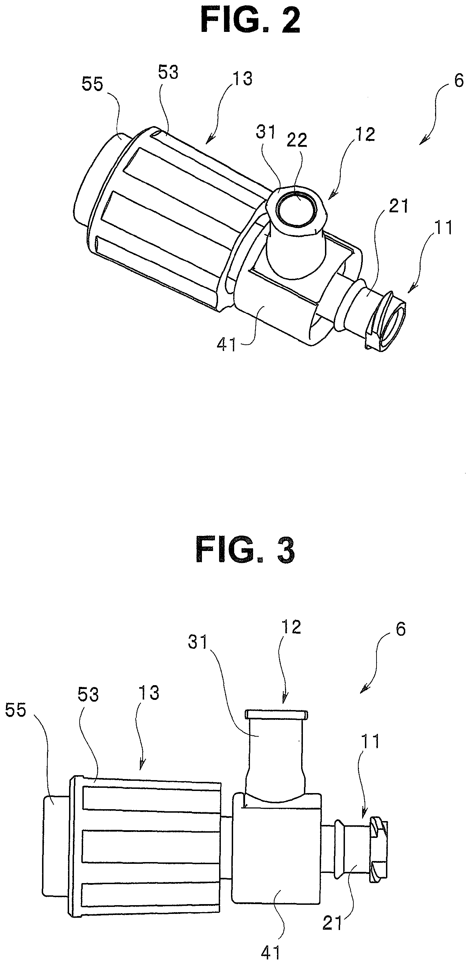

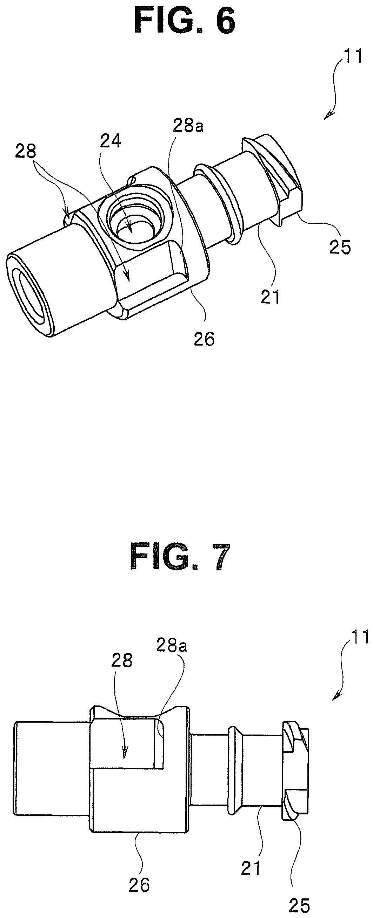

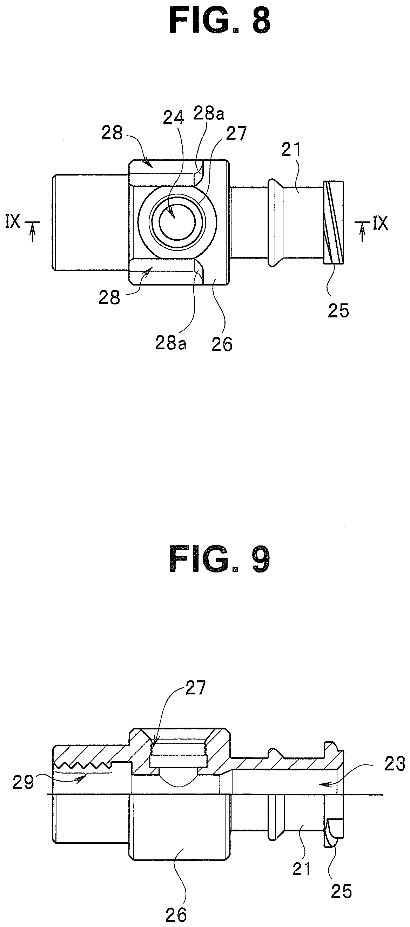

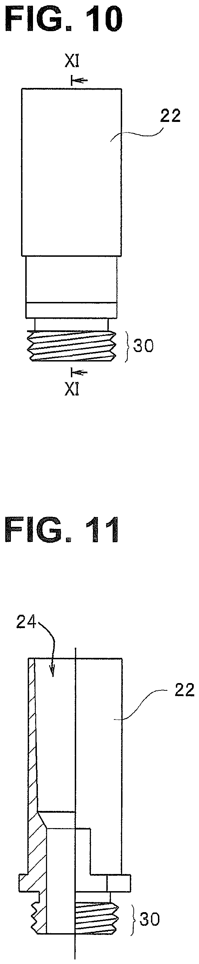

Fig. 2 is a perspective view illustrating an outer appearance of the adaptor 6. Fig. 3 is a front view illustrating the outer appearance of the adaptor 6. Fig. 4 is a half cross-sectional view along an axis direction of the adaptor 6. Fig. 5 is a cross-sectional view along line V-V in Fig. 4. Fig. 6 is a perspective view of a duct body 21 included in the port portion 11. Fig. 7 is a front view of the duct body 21. Fig. 8 is a plan view of the duct body 21. Fig. 9 is a half cross-sectional view along line IX-IX in Fig. 8. Fig. 10 is a front view of a duct member 22 included in the port portion 12. Fig. 11 is a half cross-sectional view of the duct member 22 along line XI-XI in Fig. 10.

As illustrated in Figs. 4 and 9, the port portion 11 of the adaptor 6, which includes a metal of stainless steel, includes a duct body 21 formed in a rough tubular shape. In an inner portion of the duct body 21, a treatment instrument insertion passageway 23 that longitudinally extends through the inner portion along a center axis is formed.

As illustrated in Figs. 6 and 7, the duct body 21 includes a fitting 25 at a distal end portion thereof, enabling the forceps plug 14 (Fig. 4) to be fitted thereto as described above. A proximal end portion of the duct body 21 is configured so that the proximal end portion can be attached and fixed to the endoscope attachment portion 13.

In a midway portion of the duct body 21, an increased-diameter portion 26 is formed. In the upper side of the increased-diameter portion 26, a threaded hole 27 is formed, enabling a thread portion 30 (Fig. 10), which is formed on the proximal end side of the duct member 22, to be threadably connected to the threaded hole 27. An adhesive is applied to the threaded hole 27 and the thread portion 30 (Fig. 10) of the duct member 22 is screwed into the threaded hole 27, whereby the duct member 22 is bonded and fixed to the duct body 21.

Although, here, a metal duct in the adaptor 6 includes two members, which are the duct body 21 and the duct member 22, the metal duct may include one duct member including two port portions 11 and 12. In other words, the metal duct in the adaptor 6 only needs to be a metal duct member including the first port portion 12 that communicates with the treatment instrument insertion port 5, and the second port portion 11 that communicates with the treatment instrument insertion port 5, here, the metal duct member includes the metal first duct member 22 for the port portion 12 and the duct body 21, which is a metal second duct member for the port portion 11.

At the increased-diameter portion 26, two cutout portions 28, which are as if the cutout portions 28 are obtained as a result of parts of the cylindrical increased-diameter portion 26 being shaved off. In other words, as will be described later, the metal duct member, which includes the duct body 21 and the duct member 22, includes cutout portions 28 that receive two extension portions 35.

As illustrated in Figs. 4 and 9, a thread portion 29 is formed in an inner peripheral face of the proximal end portion of the duct body 21.

The port portion 12, which also includes a metal of stainless steel, includes a duct member 22 formed in a rough tubular shape. The duct member 22 is provided so as to project laterally (upward in Figs. 4 and 5) at the midway portion of the duct body 21. In an inner portion of the duct member 22, a water feeding passageway 24 that longitudinally extends through the inner portion along a center axis and communicates with the treatment instrument insertion passageway 23 in the duct body 21 is formed.

As illustrated in Figs. 10 and 11, the thread portion 30 is formed in a part on the proximal end side of an outer peripheral portion of the duct member 22.

Then, a water feeding duct cover 31 is fitted to the duct member 22 so as to cover the outer peripheral portion of the duct member 22 with the duct member 22 fixed to the duct body 21.

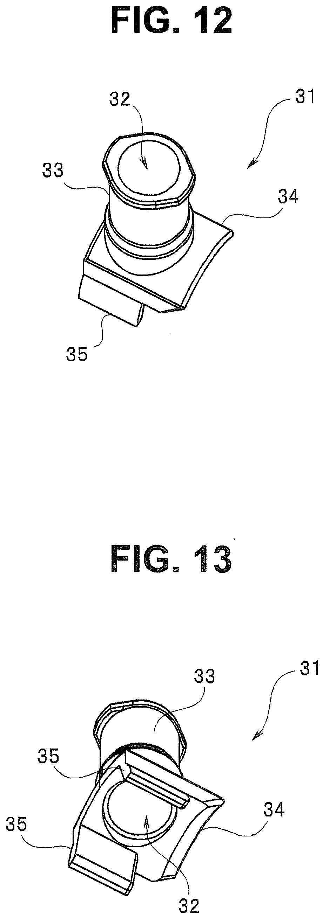

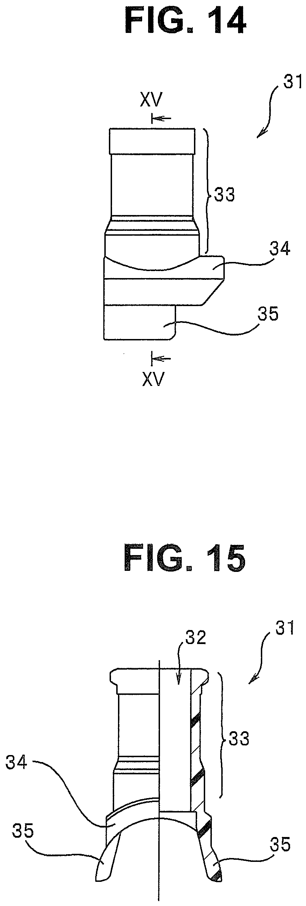

Fig. 12 is a perspective view of the water feeding duct cover 31 as viewed obliquely from the above. Fig. 13 is a perspective view of the water feeding duct cover 31 as viewed obliquely from underneath. Fig. 14 is a front view of the water feeding duct cover 31. Fig. 15 is a half cross-sectional view along line XV-XV in Fig. 14.

The water feeding duct cover 31, which is a resin member, is a cover member including, for example, polyphenylsulfone.

The water feeding duct cover 31 includes a tubular portion 33 including, inside, a duct insertion hole 32 through which the duct member 22 is inserted, a duct body cover portion 34 formed at a proximal end portion of the tubular portion 33, the duct body cover portion 34 covering a part of an outer peripheral portion of the duct body 21, and two extension portions 35 formed so as to extend to the proximal end side from the duct body cover portion 34.

As illustrated in Fig. 15, the two extension portions 35 extend from the duct body cover portion 34 in such a manner that end portions of the extension portions 35 extend away from each other toward the proximal end side. In other words, the water feeding duct cover 31 is a first sheathing member including an insulating member, the first sheathing member covering at least a part of an outer surface of the port portion 12 and including extension portions 35 that extend so as to spread toward the proximal end side.

The two extension portions 35 are formed and provided in such a manner that when the duct member 22 is inserted into the duct insertion hole 32 of the water feeding duct cover 31 and the water feeding duct cover 31 is fitted to the duct member 22 so that the duct body cover portion 34 covers the part of the outer peripheral portion of the duct body 21, the respective extension portions 35 enter the cutout portions 28.

Then, the duct body cover 41 can be fitted to the duct body 21 from the proximal end side of the duct body 21.

The duct body cover 41, which is a resin member, is a cover member including, for example, polyphenylsulfone.

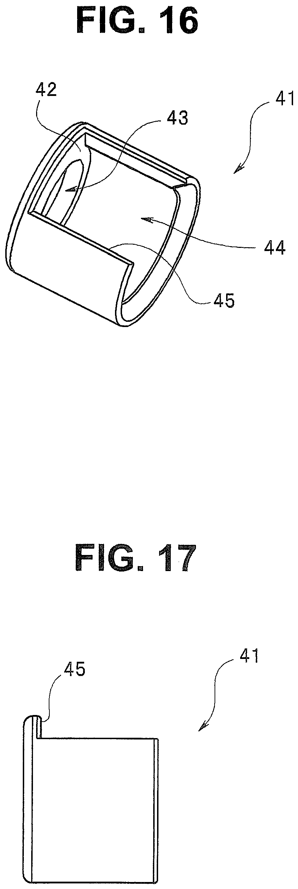

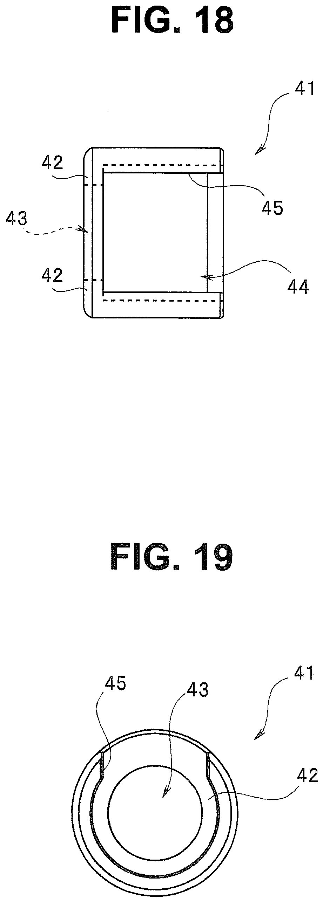

Fig. 16 is a perspective view of the duct body cover 41 as viewed obliquely from above. Fig. 16 is a front view of the duct body cover 41. Fig. 18 is a plan view of the duct body cover 41. Fig. 19 is a right side view.

On the proximal end side of the duct body cover 41, an inward flange portion 42 including, in a center portion, a hole portion 43 through which the duct body 21 is inserted is provided. In other words, the duct body cover 41 is a cylindrical member including the inward flange portion 42. On the distal end side of the duct body cover 41, a space portion 44 including an opening portion that can receive the increased-diameter portion 26 of the duct body 21 is formed and provided.

A cutout portion 45 is formed in a part of an outer peripheral wall of the duct body cover 41, and when the duct body cover 41 is fitted to the duct body 21, the water feeding duct cover 31 covering the duct member 22 projects in a radial direction via the cutout portion 45.

As will be described later, the duct body cover 41 including the insulating member provides a second sheathing member that covers at least a part of an outer surface of the port portion 11 and covers the two extension portions 35 of the water feeding duct cover 31 so as to prevent the water feeding duct cover 31 from moving to the distal end side of the port portion 12 and coming off.

As illustrated in Fig. 4, the endoscope attachment portion 13 includes a substantially-cylindrical fixing shaft member 51, a fixing thread member 52 and a cover member 53.

The stainless-steel fixing shaft member 51 includes a tapered surface 51a in an outer peripheral portion in such a manner that the tapered surface 51a has an outer diameter decreasing toward the proximal end side. The tapered surface 51a has a shape that provides a close fit with an inner peripheral face of a fitting 5a provided at the treatment instrument insertion port 5. Furthermore, in a midway portion of the fixing shaft member 51, a flange portion 51b provided so as to project in a radial direction is formed.

A stainless-steel C-shaped spring washer 54 is loosely fixed to a midway portion of the fixing shaft member 51.

The stainless-steel fixing thread member 52 includes an inward flange portion 52a on the distal end side, and in an inner peripheral face on the proximal end side, a thread portion 52b is formed. The fixing thread member 52 is provided so as to be slidable along an axis direction of the fixing shaft member 51.

The endoscope attachment portion 13 includes a skirt member 55 on the proximal end side. A distal end side part of the silicon cylindrical skirt member 55 is fixed to a proximal end portion of the fixing thread member 52 by means of insert molding. A thread portion 56 is formed at a distal end portion of the fixing shaft member 51.

The cylindrical cover member 53 is fixed to respective outer peripheral portions of the fixing thread member 52 and the skirt member 55 via an adhesive material. The cover member 53 includes an outer peripheral face in which a plurality of flat portions are formed circumferentially.

In a midway portion of the fixing thread member 52, a receiving space 52c that receives the spring washer 54 is formed is formed on the inner peripheral face side.

An adhesive is applied to the thread portion 56 of the fixing shaft member 51 and the thread portion 56 is threadably connected to the thread portion 29 at the proximal end portion of the duct body 21, whereby the fixing shaft member 51 of the endoscope attachment portion 13 is fixed to the duct body 21 with the duct body cover 41 fitted to the duct body 21.

Upon the cover member 53 being turned with the fitting 5a, which is provided to the treatment instrument insertion port 5 of the endoscope 1, inserted to the inside of the proximal end side of the skirt member 55, the fitting 5a slides along the thread portion 52b formed in the inner peripheral face of the fixing thread member 52, and the endoscope attachment portion 13 moves toward the treatment instrument insertion port 5.

When the endoscope attachment portion 13 moves toward the treatment instrument insertion port 5, the inward flange portion 52a of the fixing thread member 52 compresses the spring washer 54. A spring force of the spring washer 54 makes the fitting 5a be firmly held by the thread portion 52b of the fixing thread member 52, making the endoscope attachment portion 13 be closely attached to the fitting 5a provided to the treatment instrument insertion port 5.

Also, as illustrated in Fig. 4, the duct body cover 41 and the cover member 53 are configured so that an outer diameter L1 of a proximal end portion of the duct body cover 41 is larger than an inner diameter L2 of a distal end portion of the cover member 53 of the endoscope attachment portion 13.

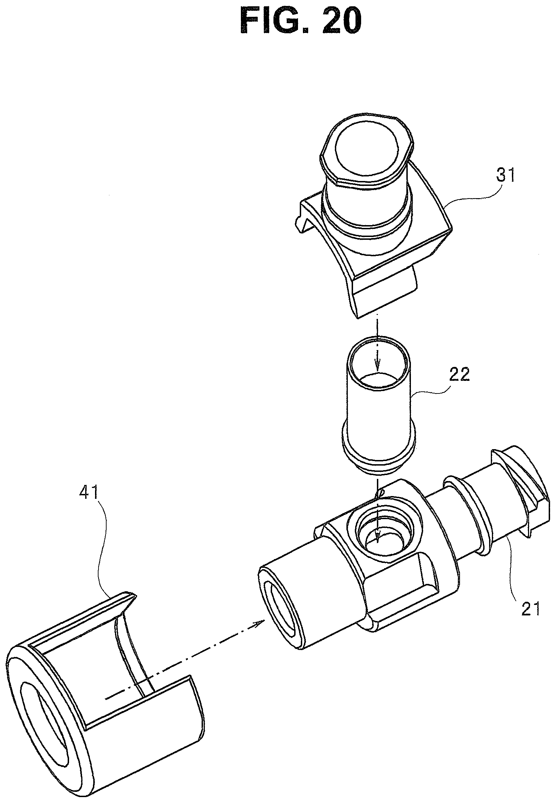

Next, assembling of the adaptor 6 will be described with reference to Fig. 20. Fig. 20 is an exploded and assembly view for describing a method for assembling the adaptor 6.

The thread portions 27 and 30 are threadably connected with an adhesive applied thereto to bond and fix the duct member 22 to the duct body 21. Subsequently, an adhesive is applied to an outer peripheral face of the duct member 22, and the water feeding duct cover 31 is put onto, and bonded and fixed to the duct member 22.

An adhesive is applied to a part of the water feeding duct cover 31 and an outer peripheral face of the duct body 21, and the duct body cover 41 is put on, and bonded and fixed to the duct body 21 and a part of the water feeding duct cover 31 from the proximal end side of the duct body 21.

Then, the thread portions 29 and 56 are threadably connected with an adhesive applied thereto, whereby the endoscope attachment portion 13 is bonded and fixed to the duct body 21.

As described above, the adaptor 6 is assembled. The adaptor 6 is fitted to the treatment instrument insertion port 5 of the endoscope 1 via the endoscope attachment portion 13.

### (Operation)

Next, an operation of the above-described adaptor 6 will be described.

In a state in which the duct member 22 is connected, and bonded and fixed to the duct body 21, the water feeding duct cover 31 is put on, and bonded and fixed to the outer surface of the duct member 22 so as to cover the outer surface. Here, the two extension portions 35 of the water feeding duct cover 31 enter the two cutout portions 28 formed in the duct body 21.

Furthermore, in a state in which the water feeding duct cover 31 is bonded and fixed to the duct member 22, the duct body cover 41 is fitted to the duct body 21 from the proximal end side of the duct body 21 so that the duct body cover portion 34 of the water feeding duct cover 31 is disposed inside the cutout portion 45 of the duct body cover 41 and the duct body cover 41 covers the two extension portions 35 and the duct body 21.

The duct body cover 41 is fitted to the duct body 21 from the proximal end side of the duct body 21 so as to cover the duct body 21 and the two extension portions 35 of the water feeding duct cover 31, and bonded and fixed to the duct body 21 and the water feeding duct cover 31. Here, as illustrated in Fig. 5, since the two extension portions 35 extend from the duct body cover portion 34 in such a manner that the two extension portions 35 extend away from each other with reference to the center axis of the duct body 21, an edge portion of the cutout portion 45 of the duct body cover 41 prevents the two extension portions 35 from coming off from the two cutout portions 28.

In other words, the duct body cover 41 provides a sheathing member including an insulating member, the sheathing member covering at least a part of the outer surface of the duct body 21 included in the port portion 11, and covering the two extension portions 35 of the water feeding duct cover 31 so as to prevent the water feeding duct cover 31 from moving to the distal end side of the duct member 22 included in the port portion 12 and coming off.

There may be cases where the duct body cover 41 comes off from the duct body 21 and the water feeding duct cover 31 and moves to the proximal end side because of adhesive deterioration.

However, even in such case, as illustrated in Fig. 4, since the outer diameter L1 of the proximal end portion of the duct body cover 41 is larger than the inner diameter L2 of the distal end portion of the cover member 53 of the endoscope attachment portion 13, the proximal end portion of the duct body cover 41 abuts to the distal end portion 53a of the cover member 53, whereby the duct body cover 41 is prevented by the cover member 53 from coming off from the duct body 21. Therefore, the endoscope attachment portion 13 includes the distal end portion 53a, which serves as a movement restricting section for preventing the duct body cover 41 from moving to the proximal end side. In other words, the endoscope attachment portion 13 includes the cover member 53, which is a cylindrical member, and the inner diameter of the cover member 53 is smaller than the outer diameter of the duct body cover 41 so as to prevent the duct body cover 41 from moving to the proximal end side of the port portion 11. Therefore, the distal end portion 53a of the cover member 53, which is a cylindrical member, provides a movement restricting section for the duct body cover 41.

Furthermore, the duct body cover 41 and the cover member 53 are formed so that in a state in which the proximal end portion of the duct body cover 41 is in abutment with the distal end portion of the cover member 53, the duct body cover 41 covers at least respective parts of the two extension portions 35, preventing the two extension portions 35 from coming off from the two cutout portions 28. In other words, even if the duct body cover 41 moves to the proximal end side because of adhesive deterioration, the duct body cover 41 still covers at least parts of the two extension portions 35 on the distal end side, the duct body cover 41 is prevented from completely coming off from the duct body 21 and the water feeding duct cover 31, and the water feeding duct cover 31 is also prevented from coming off from the duct member 22.

As a result, the covering of the duct member 22 by the water feeding duct cover 31 is maintained and the covering of the duct body 21 by the duct body cover 41 is also maintained. Therefore, a surface of the duct member 22, which is a metal member, is not exposed, and no broad area of a surface of the duct body 21, which is a metal member, is exposed.

### (Modification)

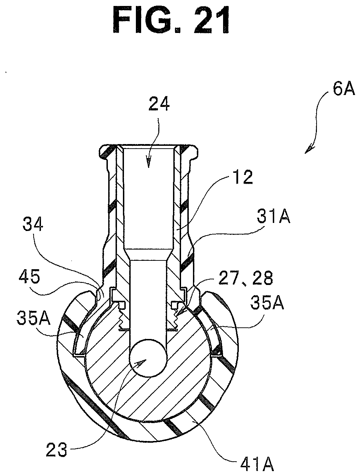

Note that although in the above example, the cutout portions 28 are formed in the duct body 21, the duct body 21 may have no cutout portions 28. A modification of an adaptor including no cutout portions 28 will be described with reference to Fig. 21.

Fig. 21 is a cross-sectional view of an adaptor 6A according to a modification of the adaptor 6 along line V-V in Fig. 4.

The duct body 21 in the adaptor 6 according to the above-described embodiment includes two cutout portions 28, and the adaptor 6 is configured so that the two extension portions 35 enter the respective cutout portions 28. As a result, the above-described adaptor 6 provides reduction in outer diameter around the axis of the duct body 21, enabling reduction in size of the adaptor.

However, a duct body 21 in the adaptor 6A according to the present modification, which is illustrated in Fig. 21, does not include the two cutout portions 28. Instead, two extension portions 35A of a water feeding duct cover 31A spread toward the proximal end side so as to cover an outer surface of a duct body 21.

Then, a duct body cover 41A is configured so as to cover the two spread extension portions 35A so that the two extension portions 35A are prevented from coming off toward an distal end direction of the water feeding duct cover 31A.

Therefore, the adaptor 6A having the configuration according to the present modification also provides effects that are similar to those of the adaptor 6 according to the above-described embodiment.

As described above, the above-described embodiment and modification provide an adaptor for endoscopes that enables ensuring safety at the time of use of high-frequency current, without decrease in strength of ducts of the adaptor themselves.

The present invention is not limited to the above-described embodiment, and various modifications, alterations and the like are possible without departing the scope of the appended claims.

### Industrial Applicability

Although the adaptors 6 and 6A have been described as examples including a water feeding port, the present invention is applicable also to an adaptor including an air feeding port.

The present application is filed claiming the priority of Japanese Patent Application No. 2013-089765 filed in Japan on April 22, 2013.

## Claims

1. An adaptor (6) for an endoscope (1), the adaptor (6) comprising:
an endoscope attachment portion (13) to be attached to a treatment instrument insertion port (5) of the endoscope (1);
a metal duct member including a duct body (21), a first port portion (12) that is adapted to communicate with the treatment instrument insertion port (5), and a second port portion (11) that is adapted to communicate with the treatment instrument insertion port (5) when the attachment portion(13) is attached to the treatment instrument insertion port (5) of the endoscope;
the first and second port portions having proximal end sides towards the duct body and distal end sides opposite to it;
a first sheathing member (31) including an insulating member, the first sheathing member (31) covering at least a part of an outer surface of the first port portion (12) and **characterized in that** the first sheathing member includes an extension portion (35) that extends so as to spread toward the proximal end side of the first port portion; and further **characterized by** a second sheathing member (41) including an insulating member, the second sheathing member (41) covering at least a part of an outer surface of the second port portion (11), and covering the extension portion (35) of the first sheathing member (31) so as to prevent the first sheathing member (31) from moving to Z the distal end side of the first port portion (12) and coming off.

2. The adaptor for an endoscope according to claim 1, wherein the endoscope attachment portion (13) includes a first movement restricting section (53a) for preventing the second sheathing member (41) from moving to the proximal end side of the second port portion (11).

3. The adaptor for an endoscope according to claim 2, wherein:
the endoscope attachment portion (13) includes a cylindrical member (53),
an inner diameter of the cylindrical member (53) is smaller than an outer diameter of the second sheathing member (41) so as to prevent the second sheathing member (41) from moving to the proximal end side of the second port portion (11), and
the first movement restricting section (53a) is a distal end portion of the cylindrical member (53).

4. The adaptor for an endoscope according to claim 1, wherein the duct member includes a metal first duct member (22) for the first port portion (12), and a metal second duct member (21) for the second port portion (11).

5. The adaptor for an endoscope according to claim 1, wherein the duct member includes a cutout portion (28) that receives the extension portion (35).

6. The adaptor for an endoscope according to claim 1, wherein the second port portion (11) is configured so that a plug member (14) including an insulating member can be attached to a distal end portion thereof.

7. The adaptor for an endoscope according to claim 1, wherein:
the endoscope attachment portion (13) includes a first movement restricting section (53a) for preventing the second sheathing member (41) from moving to a proximal end side of the second port portion (11), and a cylindrical member (53),
an inner diameter of the cylindrical member (53) is smaller than an outer diameter of the second sheathing member (41) so as to prevent the second sheathing member (41) from moving to the proximal end side of the second port portion (11),
the first movement restricting section (53a) is a distal end portion of the cylindrical member (53), and
the duct member includes a metal first duct member (22) for the first port portion (12), and a metal second duct member (21) for the second port portion (11).

8. The adaptor for an endoscope according to claim 7, wherein the second port portion (11) is configured so that a plug member (14) including an insulating member can be attached to a distal end portion thereof.

9. An endoscope (1) comprising the adaptor (6) for an endoscope (1) according to claim 1.

## Patentansprüche

1. Adapter (6) für ein Endoskop (1), wobei der Adapter (6) Folgendes umfasst:
einen Endoskopanbringungsabschnitt (13), der an einem Stutzen (5) zum Einsetzen eines Behandlungsinstruments des Endoskops (1) anzubringen ist;
ein metallisches Röhrenelement, das einen Röhrenkörper (21), einen ersten Stutzenabschnitt (12), der geeignet ist, um mit dem Stutzen (5) zum Einsetzen eines Behandlungsinstruments zu kommunizieren, und einen zweiten Stutzenabschnitt (11), der geeignet ist, um mit dem Stutzen (5) zum Einsetzen eines Behandlungsinstruments zu kommunizieren, wenn der Anbringungsabschnitt (13) an dem Stutzen (5) zum Einsetzen eines Behandlungsinstruments des Endoskops angebracht ist, umfasst;
wobei die ersten und zweiten Stutzenabschnitte proximale Endseiten in Richtung auf den Röhrenkörper und distale Endseiten diesem gegenüber aufweisen;
ein erstes Ummantelungselement (31), das ein Isolierelement umfasst, wobei das erste Ummantelungselement (31) mindestens einen Teil einer äußeren Oberfläche des ersten Stutzenabschnitts (12) bedeckt, und **dadurch gekennzeichnet, dass** das erste Ummantelungselement einen Verlängerungsabschnitt (35) umfasst, der sich verlängert, um sich in Richtung auf die proximale Endseite des ersten Stutzenabschnitts auszudehnen;
und ferner **gekennzeichnet durch** ein zweites Ummantelungselement (41), das ein Isolierelement umfasst, wobei das zweite Ummantelungselement (41) mindestens einen Teil einer äußeren Oberfläche des zweiten Stutzenabschnitts (11) bedeckt und den Verlängerungsabschnitt (35) des ersten Ummantelungsabschnitts (31) bedeckt, um zu verhindern, dass sich das erste Ummantelungselement (31) zu der distalen Endseite des ersten Stutzenabschnitts (12) bewegt und sich löst.

2. Adapter für ein Endoskop nach Anspruch 1, wobei der Endoskopanbringungsabschnitt (13) einen ersten Teil (53a) zum Einschränken einer Bewegung umfasst, um zu verhindern, dass sich das zweite Ummantelungselement (41) zu der proximalen Endseite des zweiten Stutzenabschnitts (11) bewegt.

3. Adapter für ein Endoskop nach Anspruch 2, wobei:
der Endoskopanbringungsabschnitt (13) ein zylindrisches Element (53) umfasst,
ein Innendurchmesser des zylindrischen Elements (53) kleiner als ein Außendurchmesser des zweiten Ummantelungselements (41) ist, um zu verhindern, dass sich das zweite Ummantelungselement (41) zu der proximalen Endseite des zweiten Stutzenabschnitts (11) bewegt, und
der erste Teil (53a) zum Einschränken einer Bewegung ein distaler Endabschnitt des zylindrischen Elements (53) ist.

4. Adapter für ein Endoskop nach Anspruch 1, wobei das Röhrenelement ein metallisches erstes Röhrenelement (22) für den ersten Stutzenabschnitt (12) und ein metallisches zweites Röhrenelement (21) für den zweiten Stutzenabschnitt (11) umfasst.

5. Adapter für ein Endoskop nach Anspruch 1, wobei das Röhrenelement einen Ausschnittabschnitt (28) umfasst, der den Verlängerungsabschnitt (35) aufnimmt.

6. Adapter für ein Endoskop nach Anspruch 1, wobei der zweite Stutzenabschnitt (11) derart konfiguriert ist, dass ein Verschlusselement (14), das ein Isolierelement umfasst, an einem distalen Endabschnitt desselben angebracht werden kann.

7. Adapter für ein Endoskop nach Anspruch 1, wobei:
der Endoskopanbringungsabschnitt (13) einen ersten Teil (53a) zum Einschränken einer Bewegung, um zu verhindern, dass sich das zweite Ummantelungselement (41) zu einer proximalen Endseite des zweiten Stutzenabschnitts (11) bewegt, und ein zylindrisches Element (53) umfasst,
ein Innendurchmesser des zylindrischen Elements (53) kleiner als ein Außendurchmesser des zweiten Ummantelungselements (41) ist, um zu verhindern, dass sich das zweite Ummantelungselement (41) zu der proximalen Endseite des zweiten Stutzenabschnitts (11) bewegt,
der erste Teil (53a) zum Einschränken einer Bewegung ein distaler Endabschnitt des zylindrischen Elements (53) ist, und
das Röhrenelement ein metallisches erstes Röhrenelement (22) für den ersten Stutzenabschnitt (12) und ein metallisches zweites Röhrenelement (21) für den zweiten Stutzenabschnitt (11) umfasst.

8. Adapter für ein Endoskop nach Anspruch 7, wobei der zweite Stutzenabschnitt (11) derart konfiguriert ist, dass ein Verschlusselement (14), das ein Isolierelement umfasst, an einem distalen Endabschnitt desselben angebracht werden kann.

9. Endoskop (1), umfassend den Adapter (6) für ein Endoskop (1) nach Anspruch 1.

## Revendications

1. Adaptateur (6) pour un endoscope (1), l'adaptateur (6) comprenant :
une partie d'attache d'endoscope (13) à attacher à un orifice d'insertion d'instrument de traitement (5) de l'endoscope (1) ;
un élément de conduit métallique comprenant un corps de conduit (21), une première partie orifice (12) qui est adaptée pour communiquer avec l'orifice d'insertion d'instrument de traitement (5), et une seconde partie orifice (11) qui est adaptée pour communiquer avec l'orifice d'insertion d'instrument de traitement (5) lorsque la partie d'attache (13) est attachée à l'orifice d'insertion d'instrument de traitement (5) de l'endoscope ; les première et seconde parties orifices ayant des côtés d'extrémité proximale vers le corps de conduit et des côtés d'extrémité distale opposés à celui-ci ;
un premier élément de gaine (31) comprenant un élément isolant, le premier élément de gaine (31) couvrant au moins une partie d'une surface externe de la première partie orifice (12), et **caractérisé par le fait que** le premier élément de gaine comprend une partie d'extension (35) qui se prolonge de façon à s'étendre vers le côté d'extrémité proximale de la première partie orifice ; et
**caractérisé en outre par** un second élément de gaine (41) comprenant un élément isolant, le second élément de gaine (41) couvrant au moins une partie d'une surface externe de la seconde partie orifice (11), et couvrant la partie d'extension (35) du premier élément de gaine (31) de façon à empêcher le premier élément de gaine (31) de se déplacer vers le côté d'extrémité distale de la première partie orifice (12) et de se détacher.

2. Adaptateur pour un endoscope selon la revendication 1, dans lequel la partie d'attache d'endoscope (13) comprend une première section de restriction de mouvement (53a) pour empêcher le second élément de gaine (41) de se déplacer vers le côté d'extrémité proximale de la seconde partie orifice (11).

3. Adaptateur pour un endoscope selon la revendication 2, dans lequel :
la partie d'attache d'endoscope (13) comprend un élément cylindrique (53),
un diamètre interne de l'élément cylindrique (53) est plus petit qu'un diamètre externe du second élément de gaine (41) de façon à empêcher le second élément de gaine (41) de se déplacer vers le côté d'extrémité proximale de la seconde partie orifice (11), et
la première section de restriction de mouvement (53a) est une partie d'extrémité distale de l'élément cylindrique (53).

4. Adaptateur pour un endoscope selon la revendication 1, dans lequel l'élément de conduit comprend un premier élément de conduit métallique (22) pour la première partie orifice (12), et un second élément de conduit métallique (21) pour la seconde partie orifice (11).

5. Adaptateur pour un endoscope selon la revendication 1, dans lequel l'élément de conduit comprend une partie de découpe (28) qui reçoit la partie d'extension (35).

6. Adaptateur pour un endoscope selon la revendication 1, dans lequel la seconde partie orifice (11) est configurée de telle sorte qu'un élément bouchon (14) comprenant un élément isolant peut être attaché à une partie d'extrémité distale de celle-ci.

7. Adaptateur pour un endoscope selon la revendication 1, dans lequel :
la partie d'attache d'endoscope (13) comprend une première section de restriction de mouvement (53a) pour empêcher le second élément de gaine (41) de se déplacer vers un côté d'extrémité proximale de la seconde partie orifice (11), et un élément cylindrique (53),
un diamètre interne de l'élément cylindrique (53) est plus petit qu'un diamètre externe du second élément de gaine (41) de façon à empêcher le second élément de gaine (41) de se déplacer vers le côté d'extrémité proximale de la seconde partie orifice (11),
la première section de restriction de mouvement (53a) est une partie d'extrémité distale de l'élément cylindrique (53), et
l'élément de conduit comprend un premier élément de conduit métallique (22) pour la première partie orifice (12), et un second élément de conduit métallique (21) pour la seconde partie orifice (11).

8. Adaptateur pour un endoscope selon la revendication 7, dans lequel la seconde partie orifice (11) est configurée de telle sorte qu'un élément bouchon (14) comprenant un élément isolant peut être attaché à une partie d'extrémité distale de celle-ci.

9. Endoscope (1) comprenant l'adaptateur (6) pour un endoscope (1) selon la revendication 1.
